# EUROPEAN PATENT APPLICATION

(11) **EP 1 471 070 A1**
(43) Date of publication of application: **27.10.2004**
(21) Application number: 03076175.3
(22) Date of filing: 22.04.2003
(51) Int. Cl.: C07G 1/00, A23L 1/308, A61K 7/00

(54) **Method for recovering a secondary plant metabolite**

(71) Applicant: Royal Schouten Group N.V., 4283 GG Giessen (NL)
(72) Inventor: Verbruggen, Maria Anna, 6871 ZS Renkun (NL); Bakker, Rudolf Wilhelmus Maria, 2411 ZB Bodegraven (NL)
(74) Representative: Prins, Adrianus Willem, Mr. Ir.

(57) **Abstract**

The present invention relates to a method for recovering a secondary plant metabolite from a seed material,. The present invention further relates to a extract comprising the secondary plant metabolite, which extract is obtainable by a method according to the invention and to a foodstuff, cosmetic or medicament comprising such an extract.

## Description

The invention relates to a method for recovering a phytochemical, more in particular a secondary plant metabolite, from a seed material.

Phytochemicals are secondary plant metabolites, that are compounds typical or even unique to a specific group of plants. Many of these secondary plant metabolites can chemically be characterized as phenolics, having a hydroxylated aromatic structure in common.

Phytochemicals, such as the phenolics, have been reported to be of potential use in food and pharmaceutical applications. For example, lignans, a group of phenolics, are considered to exhibit a broad spectrum of biological activities such as anti-tumour, anti-mitotic, antioxidant, antiviral, weak estrogenic and anti-estrogenic activities. Lignans are ubiquitously present in plant materials, such as in seeds, for example in linseed.

Even though there would appear to be very significant commercial uses for lignans, such as SDG, in foods and medicines, the greatest problem has been the low availability. In order to address this US 5,705,618 proposes a relatively simple method to extract lignans and/or cinnamic acid derivatives from flax seed by contacting an oil-free flax seed meal with an aliphatic alcohol solvent.

There remains however a need for alternative, commercially attractive ways to obtain phytochemicals such as phenolics and in particular lignans.

It has now been found that it is possible to obtain a phytochemical such as a phenolic and in particular a lignan with a good yield and in a cost-effective way from a specific part of seeds (viz. the seed hull comprising said phytochemical). Accordingly, the present invention relates to a method for recovering a phytochemical, such as a phenolic, and in particular a lignan from hulls of a seed, which method comprises fractionating a seed material comprising the hulls, thereby obtaining a fraction enriched in hulls and optionally extracting the phytochemical from said fraction.

The inventors have found that the hulls of a seed form a particular suitable source for phytochemicals such as lignans. The phytochemicals can be extracted from the hulls in an economically attractive way with a satisfactory concentration (*e.g.* more than 25 wt. % based upon the total weight of the dried extract) and good yield. An extract obtainable in accordance with the present invention may be used without further purification or concentration of the phytochemical or phytochemicals of interest. If desired, further purification and/or concentration may be performed. Suitable techniques are known in the art.

In particular, the term phytochemical is used herein to describe any secondary plant metabolite, more in particular such metabolite of phenolic nature. Preferred phytochemicals in accordance with the invention are phytochemicals that have a biological functionality in the plant such as colouring agents or compounds formed for protection, for example compounds contributing to drought resistance or disease resistance. Preferred metabolites with a biological function are often present in the outer layers of plant materials such as in the hulls or skins of seeds.

Further, phytochemicals with suggested therapeutic or disease prevention functionalities such as anti-oxidant activity or estrogen receptor binding affinity are preferred. The compounds are usually soluble in a polar solvent such as water, a C₁-C₄ alcohol or a mixture thereof. Once solubilised they generally show absorption of light in the UV-range (180-380nm)

Preferred examples of phytochemicals that may be recovered in accordance with the invention include phenolics and in particular flavonoids, lignans and stilbenes.

The term phenolics is used herein to describe molecules having at least one hydroxylated aromatic ring structure. Phenolics can be divided in many structurally related groups, such as coumarins, lignans, stilbenes, flavanoids and flavonoids. Subclasses of flavonoids are based on variations in the heterocyclic carbon-ring and include flavones, flavonols, flavanones, flavanols, anthocyanidins and isoflavones. Representatives of such compounds include the stilbene t-resveratrol, neolignans (as a subgroup of lignans), and catechins, quercetin and its derivatives, naringenin and its derivatives and tannins as examples of flavonoids.

The term lignan is used herein to describe a dimer containing a dibenzylbutane skeleton or a 1,4-diarylbutane skeleton, including complexed or otherwise bound forms thereof, such as lignans that are releasable by base-catalysed hydrolysis such as the phenolics referred to in US 5,705,618.

Preferred lignans include hydroxymatairesinol, isolariciresinol, lariciresinol, syringaresinol, secoisolariciresinol (SECO), secoisolariciresinol diglucoside (SDG) and functional analogues thereof such as matairesinol, pinoresinol, sesamin and sesamolin (including their derivatives).

The term seed material is used herein to describe a material (mainly) comprising seeds or parts thereof (such as hulls and kernels, including parts thereof). Preferably the seed material essentially consists of such seeds or parts thereof. As a seed material, any seed or hull-containing part of a seed of which the hulls contain the phytochemical of interest, such as a lignan, may be used as a source. The seed material may be of a natural occurring plant (including a wild-type variant) or of a genetically modified plant.

Particularly suitable seeds for providing the seed material include seed materials selected from the group consisting of the *Linaceae,* in particular *Linum usitatissimum* L. (linseed and flax seed), the *Poaceae,* in particular *Hordeum vulgare* L. (barley), *Zea mays* L. (corn), *Avena sativa* L. (oats), *Oryza sativa* L. (rice), *Secale cereale* L. (rye) and *Triticum aestivum* L. (wheat (durum)), the *Brassicaceae,* in particular *Brassica napus* L. (canola), *Brassica rapa* L. - *e.g.* subspecies *oleifera -* (rapeseed), *Sinapis alba* L. (white mustard seed) and *Brassica nigra* (black mustard seed), the *Arecaceae,* in particular *Cocos nucifera* L. (copra), *Elaeis guineensis* Jacq. (palm kernel), the *Malvaceae,* in particular *Gossypium hirsutum* L. (cottonseed), the *Juglandaceae,* in particular *Juglans regia* L. (groundnut), the *Papilionaceae,* in particular *Arachis hypogaea* L. (peanut), *Pisum sativum* L. (pea), *Glycine max* L. (soybean), ), *Phaseolus vulgaris* L. (beans) and *Lupinus Luteus* L. (lupine), the *Asteraceae,* in particular *Carthamus tinctorius* L. (safflower) and *Helianthus anuus* L. (sunflower seed), the *Pedaliaceae,* in particular *Sesamum indicum* L. (sesames), the *Rosaceae,* in particular *Prunus dulcis* (almond), the *Apiaceae,* in particular *Carum carvi* L. (caraway) and the *Anacardiaceae,* in particular *Mangifera indica* L. (mango).

Very good results have been achieved with seed material of the *Brassica* group,the *Pedaliaceae* group and with seed material of the *Linaceae* botanical group, in particular with seed material of linseed.

Linseed, sesames, peanut, rye, rapeseed, mustard seed and canola seed are particularly preferred.

The term hull is used herein to describe a part of the seed generally covering the seed. Encompassed within the term hull are in particular the seed coat, the skin, the husk, the pericarp, the testa and the shell of the seed.

Particular suitable as a seed material is a hull-containing meal, such as a coarse meal, extracted meal, pressed cake or oil meal.

An interesting aspect of the present invention is that as a seed material, a material can be used that is generally considered to be a waste material or a by-product, *e.g.* the defatted seed material formed from a seed used for oil recovery (such as a defatted coarse meal). Thus such a seed material is a potentially cheap source for a phytochemical, in particular for a phenolic such as a lignan. The seed material may have been defatted in any way suitable for recovering oil from the seed, such as by pressing, by extraction (*e.g.* liquid extraction with an apolar solvent such as an alkane or by CO₂ extraction) or a combination thereof. In fact, it has been found very advantageous with respect to the lignan recovery, to use a seed material that has been defatted by liquid extraction of oil. The defatting may be performed prior to fractionation of the seed material or thereafter on a hull-enriched fraction. For practical reasons, it is preferred to perform defatting prior to the fractionation of the hull-enriched fraction from the seed material.

The seed material is fractionated in one or more fractionation steps. It has been found advantageous with respect to the recovery efficiency and/or hull content in the hull-enriched fraction to liberate or release the hulls in the seed material before fractionating the material or in between two fractionation steps. Such release or liberation can suitably be achieved by subjecting the seed material to shear force such as by rollermilling and/or by impact force, *e.g.* by hammermilling or ripple-milling.

This has been found to contribute to separating the hulls from the kernels. Rollermilling has been found to be particular suitable to release or liberate the hulls. Suitable conditions depend upon the type of the seed material and the dimensions of the seeds present therein. Suitable conditions can be determined based upon general common knowledge and the present description and/or claims.

A method according to the invention may encompass one or more fractionating steps wherein the material is fractionated based upon particle size. Suitable ways of such fractionating are known in the art and include screening and sieving. Suitable conditions depend upon the type of material, in particular the size and the shape of the hulls. In practice, the conditions, such as sieve size and sieve geometry in the case of sieving, will suitably be chosen to separate the hulls from smaller parts in the material and optionally from larger parts in the material. The skilled person will know how to choose suitable conditions in order to obtain a fraction enriched in hulls with a satisfactory hull content and recovery. Good results have for example been achieved with a fraction, such as a fraction from linseed material or a material with a similar geometry, that substantially retains on a sieve having a round mesh of about 1 mm (or equivalent mesh) and that preferably substantially passes through a sieve having a round mesh of about 2.5 mm (or equivalent mesh). Preferably the fraction substantially passes on a sieve having a split mesh of about 1.5 mm (or equivalent mesh).

Very good results have been achieved with a fraction retaining on a 1 mm round mesh (or equivalent mesh) and passing through a 2 mm round mesh (or equivalent mesh).

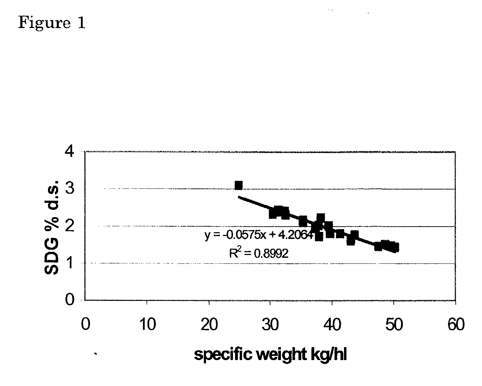

Preferably a method according to the invention comprises a fractionation based upon specific weight and/or upon geometry (in particular specific surface), such as a fractionation by a specific gravity table, a wind sifter or a combination of one or more of these devices. It has been found by the inventors that a correlation exist between the specific weight of a fraction enriched in hulls and the content of certain phytochemicals, such as phenolics and in particular of the lignan content of that fraction. An example thereof is illustrated by Figure 1, which shows the results of experiments wherein the lignan content was determined in view of the specific weight of a linseed material fraction.

The skilled person will know how to choose suitable conditions in order to obtain a fraction enriched in hulls with a satisfactory hull content and recovery, based upon common general knowledge and a routine calibration of the used fractionation system, for a specific type of material. In general, a relatively low specific weight is a good indication for a fraction with a relatively high hull content. Good results have for example been achieved with a fractionation based upon specific weight wherein a fraction is identified with a specific weight between 20 and 50 kg/hl, preferably in the range of 25-38 kg/hl, more preferably in the range of 25-30 kg/hl which fraction can then be used for further fractionation or directly for extraction of the phytochemical, in particular a phenolic such as a lignan. In particular, these values have been found to be applicable to linseed material and the like.

Preferably a combination of fractionation steps is carried out. For example, good results have been achieved with a particle size based fractionation (in particular on a sieve), followed by further fractionation of the isolated fraction (such as the fraction retaining on a sieve with >1 mm round mesh or equivalent) based upon specific weight, in particular on a specific gravity table or a wind sifter. The use of a wind sifter has been found particular advantageous with respect to the combination of a specific weight and specific surface based separation, achievable thereby.

For separating the hull fraction from remaining larger particles (e.g. straws) a fraction may be passed through a relatively high mesh sieve (e.g. 2.5 mm round mesh or equivalent) before or after a specific weight fractionation.

A fraction enriched in hulls can be identified (selected) in any way, during method development (including optimisation) and/or in practice. A skilled person will be able to take care of this, based upon common general knowledge and the information disclosed in the present description and/or claims.

The inventors further have found that a correlation exists between the colour of a hull and the content of certain phytochemicals, such as the lignan content, in particular for a hull of linseed. For obtaining a fraction with hulls with a relatively high content of the phytochemical of interest (such as the lignan content), compared to the average content of that phytochemical in the total hull content of a seed material it is therefore preferred to identify a fraction enriched in hulls based upon a colour evaluation. A desired colour depends upon the seed material. A suitable evaluation system can be defined by routine practice.

Fine-tuning of the fractionation process of hulls may be performed by colour identification, visually by eye or automated by means of a colour detection or colour recognition system. In particular for phenolics such as lignans, brown and dark separate hulls and hull parts are highly preferred, while a light or white/yellow-grey colour is an indication of lignan-poor hulls and/or of seed parts attached to the hulls.

The colour of an object is determined by the wavelength of light reflected from it's surface. In biological materials, the light reflected varies widely as a function of wavelength. These spectral variations can be used in non-destructive automated vision or colour detection/recognition systems to identify agricultural products, and for continuous classification and production monitoring of these products. Other parameters that are important in this respect are low vs. high imaging resolution, size/tolerance of hulls/hull parts and of hulls low in purity, colour vs. black & white, and speed.

Assisted by lighting, light signals are registered by colour cameras and processed in the classification software. This software uses thresholds and wavelength ranges to distinguish between brown, dark hulls (high in purity) preferably corresponding with wavelengths in the range of 700-800 nm, and more preferably in the range of 730 to 770 nm, and other hulls and seed parts which are lower in purity.

With respect to visual assessment by the eye, a skilled person will be able to visually identify a fraction with a suitable colour (*i.e.* a fraction with a relatively dark fraction for a high hull purity and/or high lignan content), based upon common knowledge and routine practice.

Alternatively or in addition a skilled person may routinely identify a suitable fraction by microscopy, by identifying a fraction wherein the hulls generally have a relatively low amount of residual kernel material attached to the hulls. Evaluation by microscopy may very suitably be performed by separation of samples by hand and evaluation by colour and by shape and size (geometry), preferably in duplicate, and calculation of the purity of the hulls on weight basis. Brown and dark separate hulls and hull parts are much preferred, while a light or white/yellow-grey colour is an indication of lignan-poor hulls and/or seed parts attached to the hulls.

Also shape and size are important factors in the microscopic evaluation of the purity of linseed hulls. Hulls with a high purity that are rich in lignans are relatively easy to identify as they are present as separate concave, soft, thin hulls, that are stripped of other seed parts, and are low in density. Hulls and seeds parts that are low in purity can be recognized by their more solid, hard, dense, and spherical character.

Good results have *inter alia* been achieved with a fraction wherein at least 50 % of the hulls is essentially free of kernel material.

The present invention allows the identification of a fraction enriched in hulls, that can be used for extracting a phytochemical, such as a phenolic and more in particular a lignan. Preferably the content of hulls, essentially free of kernel material, in an identified fraction is 50-100 wt. % based upon the total weight of the fraction, more preferably 60-100 wt. % based upon the total weight. For practical reasons the hull content is preferably up to 95 wt. %, more preferably up to 90 wt. %.

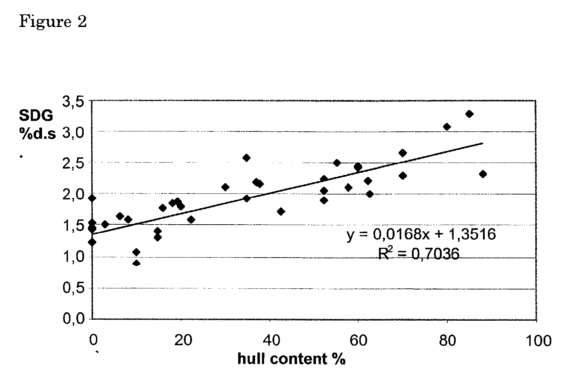

In an aspect of the invention it has been found that a fraction enriched in hulls has a high content of a phytochemical, such as a lignan, in comparison to the content of that phytochemical of the plant material from which the fraction is obtainable. It has for example been possible to obtain a fraction, wherein the concentration of a phytochemical, in particular a phenolic, is increased by more than 10 %, preferably 20-1000 %, more preferably 30-200 % compared to the seed material from which the fraction is obtained (calculated as 100 % x [concentration in fraction]/[concentration in seed material] - 100 %). Figure 2 shows results of experiments wherein the content of a lignan (SDG) was determined in view of the hull-content in the fraction.

With respect to lignans, an increase in content (based upon wt. % dry substance) with 0.2 percentile points or more, more in particular an increase with about 0.5 to up to about 2.5 percentile points has been found feasible. In absolute terms, the content of lignans in a fraction enriched in hulls depends upon the nature of the material. Typically, in particular in the case of a linseed material as a seed material (such as linseed defatted meal), a preferred fraction enriched in hulls has a lignan content of more than 1.5 wt. % based upon the dry substance, preferably of more than 1.7 wt. % based upon the dry substance, more preferably of at least 2 wt. % based upon the dry substance, even more preferably of at least 2.2 wt % based upon the dry substance. In particular in the case of linseed material (such as linseed (defatted) meal), a lignan content of about 4 wt. % or more in the hull-enriched fraction has been found feasible, although very good results have been achieved with a fraction having a lignan content of up to 3.5 % or up to 3 %. By comparison, the overall lignan content in linseed has been found to be in the range of typically 0.3-0.6 wt. %.

With respect to the lipid content in the hull fraction, the hull fraction is preferably defatted and preferably comprises less than 20 wt. % lipids, more preferably less than 15 wt. % lipids, *e.g.* about 3-10 wt. % lipids. By comparison, non-defatted linseed typically comprises 40 % or more lipids.

The extraction of the phytochemical can be performed in any suitable way to recover the phytochemicals from the fraction enriched in hulls. Suitable recovery processes have been described in the art, e.g. in US 5,705,618 and the references cited therein a number of suitable procedures for the recovery of lignans are mentioned.

For practical and economic reasons a liquid extraction is preferred. Particularly suitable extraction media include polar solvents in particular solvents comprising an aliphatic alcohol (typically at least 50 wt. %), such as a C₁-C₄ alcohol, and optionally water (typically up to 50 wt. %). Although it is possible to further purify the phytochemical of interest (such as the lignan) or to concentrate the extract, the extract may very suitably be employed as such.

The solvent may be evaporated to obtain a residue comprising the phytochemical of interest, such as the lignan. Further purification may be performed, *e.g.* by extraction and/or chromatography. The extract (optionally after further purification and/or concentration) may be chemically, physically or enzymatically processed, *e.g.* to liberate lignan, that is present in a complexed or otherwise bound form. Such processes are known in the art, e.g. from US 5,705,618.

As indicated above the present invention makes available a fraction enriched in hulls with interesting prospects for recovering a phytochemical, in particular a phenolic and more in particular a lignan. Accordingly, the present invention further relates to a fraction enriched in hulls, obtainable by a method according as described in the present description and/or claims. In particular the invention further relates to such as fraction, preferably from linseed, having a content of a phytochemical of interest, such as a lignan content, and/or a specific weight as specified above.

The present invention further relates to an extract, preferably a lignan extract, obtainable by a method as described in the present description and/or claims.

Of an extract according to the invention, it has been found that it has a relatively low carbohydrate to phytochemical of interest ratio in comparison to extracts of plant material obtainable by a conventional method such as by extraction from whole seeds or defatted meal by aqueous ethanol.

For indicative purposes, the following table shows some estimated lignan, carbohydrate and lipid weight contents of extracts, obtained by different extraction methodologies (contents are based upon dry weight).

| **Extract :** | **lignan** | **carbohydrates** | **lignan/ carbohydrates** | **lipids** |
|---|---|---|---|---|
| obt. from defatted linseed meal; aqueous ethanol extract | < 10% | > 10% | >1 | Trace |
| obt. from linseed / aqueous ethanol | < 10% | > 5% | >0.5 | Substantial |
| obt. from defatted linseed meal, extracted by aqueous ethanol, then ultrafiltrated | > 35% | < 5% | <0.14 | Trace |
| an extract of the present invention | 10-50% | > 10% | 0.2-1 (pref.) | Trace |

A preferred extract according to the invention comprises 10-50 wt. %, based upon the dry weight, of the phytochemical, such as the lignan, and at least one carbohydrate, wherein the total carbohydrate to phytochemical weight to weight ratio is at least 0.2. Much preferred is an extract wherein the total carbohydrate to phytochemical ratio is 0.2 to 1.

A preferred extract according to the invention comprises (i) a solvent selected from the group consisting of water, methanol, ethanol, propanols, butanols and mixtures comprising at least one of these solvents and (ii) a lignan - in particular SDG - or a number of lignans in a total lignan concentration of 10-50 wt.% , preferably 20-40 wt. % based upon the dry weight of the extract.

Preferably the solvent is water, ethanol or a mixture thereof, more preferably a mixture comprising at least 50 % ethanol (volume to volume).

A phytochemical - in particular a phenolic such as a lignan - an extract or a hull fraction obtainable according to a method as described in the present description and/or claims has been found very suitable for use in a product for topical or oral administration.

A phytochemical, an extract or a hull fraction obtainable in accordance with the invention has been found particularly suitable for use as or in a nutraceutical, a cosmeceutical, a foodstuff, an animal feed or a medicament.

The term nutraceutical is used herein to describe an ingredient for a food supplement or a foodstuff, said ingredient having a positive effect on the physical or psychological health of the consumer of the ingredient.

The term cosmeceutical is used herein to describe an ingredient for a cosmetic, body care or hair care product having a positive effect on the physical condition of the body (e.g. the skin or hair).

The present invention will further be illustrated by the following examples.

### Example 1

Unmilled defatted coarse meal (linseed; 1.2 wt. % SDG based upon dry substance) having a specific weight of 48 kg/hl was sieved for about 6 hours, first over a 1.5 mm split mesh, after which the passed fraction was further sieved over a 1 mm round mesh sieve of a Damas Vibam 1013 sieving machine. The fraction retaining on the 1 mm round mesh was further fractionated with a specific gravity table (SG1) (Kipp-Kelly Specific Gravity Separator SY 300) for about 11 hours, with the following settings.

| | Y | X | W | T |
|---|---|---|---|---|
| Example 1.1 | 3 | 2 | 2 | 11 |
| wherein: Y= angle front/behind (cm height difference) X= angle left/right (cm height difference) W= wind; number of turns by which inlet is opened T= rotation number (cm) | | | | |

The fraction from 37-50 cm (as measured at the large side of the triangular bed) in the 178 cm bed was identified as the hull enriched fraction (H1) of interest; the fraction from 78-111 cm (R1) was returned to the inlet of SG2. The remaining fractions - dust (S) and grit (G) - were not further processed.

Optionally the fraction was further fractionated in a second specific gravity table (SG2) (for about 5 hours) with the following settings.

| | Y | X | W | T |
|---|---|---|---|---|
| Example | 1.5 | 1 | 2 | 19 |

A hull enriched fraction (H2) was identified in the bed from 32-128 cm. The remaining fractions (dust and grit) were not further processed.

### Example 2

A coarse linseed meal (defatted, 1.4 wt. % SDG based upon dry substance, specific weight of 45 kg/hl ) was milled with a Bühler four-roller mill with the following settings:

| | |
|---|---|
| Differential | 1.2 |
| corrugations (number/cm) | 6.7 |
| edge (degrees) | 8 |
| back angle (degrees) | 65 |
| cutting angle (degrees) | 25 |
| distance between corrugations (mm) | 0.65 |

The milled meal was then sieved during about 2 hours (Ex 2.1) or about 3 hours (Ex 2.2) over a 1.0 mm round mesh sieve. The retained fraction was fractionated in a first specific gravity table (SG1) with the following settings, for about 15 min (Ex 2.1) or about 7 hours (Ex. 2.2).

| | duration | Y | X | W | T |
|---|---|---|---|---|---|
| Ex. 2.1 | 15 min | 4 | 3 | 3 | 21 |
| Ex. 2.2 | 7 hours | 4 | 3 | 3 | 19 |

In both examples a good distribution was achieved. In a total bed of 178 cm a hull enriched fraction was identified (from 0-20 cm in Example 1.1 and from 0-25 cm in Example 1.2), the fraction from 20-72 cm respectively 25-100 cm was re-fed to the inlet of the specific gravity table. The remaining fractions were not further processed.

Optionally the hull enriched fraction was further treated for about 40 min (Ex. 2.3) or about 3 hours (Ex. 2.4) in a second specific gravity table (SG2) having the following settings:

| | inlet | duration | Y | X | W | T |
|---|---|---|---|---|---|---|
| Ex. 2.3 | fraction of 2.1 | 40 min | 1 | 1 | 4 | 20 |
| Ex. 2.4 | fraction of 2.2 | 3.2 hours | 1 | 1 | 4.5 | 20 |

### Example 3

A defatted coarse linseed meal (1.4 wt. % SDG based upon dry substance, specific weight of 45 kg/hl) was milled as described in Example 2 and plansifted in a Bühler Twin Rotostar. The sifter was equipped with a 420 µm wired sieve and a 1.5 mm wired sieve. The fraction retained on the 420 µm sieve but passing through the 1.5 mm was used for further fractionation in a wind sifter (Bückmann Zig Zag Separator ZN15-80x120) with a coarse meal dosage (D) to air dosage (L) of 190 to 60 and a inlet capacity of 8 kg/hr.

### Results for Examples 1-3

| | Specific weight. (kg/hl) | Recovery of hull enriched fraction (coarse meal = 100%) | Hull content (%) | SDG recovery efficiency* (%) | SDG (%ds) | Capacity (kg/h) |
|---|---|---|---|---|---|---|
| Ex 1.1 coarse meal → sieving →SG 1 | 38 | 14 | 63 | 9 | 2,0 | 30 |
| Ex 1.2 coarse meal→ sieving → SG 1→SG 2 | 33 | 7 | 70 | 5 | 2,2 | 30 |
| Ex 2.1 coarse meal → milling → sieving → SG 1 | 31 | 13 | 61 | 8 | 2,4 | 20 |
| Ex 2.3 coarse meal→ milling→ sieving → SG 1 → SG 2 | 35 | 26 | 53 | 14 | 2,0 | 20 |
| Ex 3. 1 coarse meal → milling → sieving → wind sifting | 35 | 33 | 51 | 17 | 2.0^{x} | 4 |

| | | | | | | |
|---|---|---|---|---|---|---|
| all percentages are wt. % * Recovery of hull enriched fraction multiplied by hull-content in fraction. | | | | | | |
| ^{x} Calculated on basis of hectoliter weight | | | | | | |

The results show that the SDG content in a fraction enriched in hulls can be increased considerably in comparison to the SDG content in coarse meal. It is further shown that milling helps to improve the SDG recovery efficiency, in particular in case further fractionation is carried out in at least two specific gravity tables or with a wind sifter.

### Example 4

Coarse linseed meal with a specific weight of 48 kg/hl and a lignan content of 1.5 wt. % based upon dry substance was fractionated under the following conditions. First the material was sieved to obtain a fraction retaining on 1 mm round mesh and passing through 1.5 mm round mesh. That fraction was further fractionated on three specific gravity tables. The settings of the tables were as follows:

| | Y | X | W | T |
|---|---|---|---|---|
| SG1 | 3 | 2 | 2 | 11 |
| SG2 | 2 | 1 | 4 | 19 |
| SG3 | 2.5 | 1 | 4 | 17 |

Various fractions were taken to obtain fractions differing in hull-content and fractions differing in specific weight. Of these fractions the lignan content was determined. Figures 1 and 2 show the results, which indicate a relationship between specific weight respectively the hull-content in the fraction on the one hand and the lignan content on the other hand.

### Example 5

Linseeds (10.2% moisture) where dried for 20-45 min to 2.8-4.4% moisture in a fluid bed drier (air temperature 106 °C). After cooling for 30 min, the seeds where fed into a ripple mill (CPM, type 8/6) with a rotor speed of 3000 rpm and with an intake of 100-130 kg linseed/h. Intact seeds, endosperm, seed parts and hulls where subsequently separated with a specific gravity table (Damas, LAKTA gravity separator) in 29% hulls, 52% endosperm and 19% intact seeds based on weight percentage.

Hulls of dried peas and dried lupine seeds were also successfully obtained from seeds by treatment with the ripple mill and subsequent fractionation in hulls and in intact and broken seeds with the specific gravity table.

Hulls from undried sesame seeds were obtained by ripple milling and subsequent fractionation of intact seeds, endosperm and seed parts with the specific gravity table to yield in 25% hulls by weight.

### Example 6

Before fractionation, seed materials were treated as follows:

Soy beans (moisture 8.9%) were dried for 30 min at 108 °C to 4.7% moisture in a fluid bed drier and after cooling in ambient air broken with a pea splitter (set-screw 9 mm, 250 rpm).

Lupine seeds were dried for 45 min at 110 °C to 6.3% moisture in a fluid bed drier and after cooling in ambient air, broken with the pea splitter (set-screw 9 mm, 250 rpm).

Cranberry beans (2000 crop, moisture 17.0%) were dried for 45 min at 110 °C to 11.2% moisture in a fluid bed drier, cooled in ambient air and broken with a pea splitter (set-screw 12 mm, 250 rpm).

White fodder beans (2000 crop, moisture 17.4%) where dried for 40 min at 110 °C to 9.0% moisture in a fluid bed drier, cooled in ambient air and broken with a pea splitter (set-screw 9 mm, 250 rpm).

White beans (Manitoba, November '99) were dried for 20 min at 110 °C to 10.4% moisture in a fluid bed drier, cooled in ambient air and broken with the pea splitter (set-screw 9 mm, 250 rpm).

Pinto beans (2000 crop, moisture 15.3%) were dried in a fluid bed drier for 45 min at 105 °C to 9.2% moisture, cooled in ambient air and broken with a pea splitter (set-screw 12 mm, 250 rpm).

Dutch brown beans (crop 2000) where dried in a fluid bed drier for 30 min at 110 °C to 8.2% moisture, cooled in ambient air and broken with the pea splitter (set-screw 12 mm, 250 rpm).

Dark Red Kidney beans (Ontario origin, 2000 crop) were dried in a fluid bed drier for 40 min at 110 °C to 7.7.% moisture, cooled in ambient air and broken with the pea splitter (set-screw 12 mm, 250 rpm).

Peas with a moisture content of 13.6% were dried in a fluid bed drier to 6.5% moisture in a fluid bed, cooled in ambient air and broken with the pea splitter (250 rpm).

After treatment with the pea splitter each of the seed materials were subsequently subjected to two separating steps by fractionation on the specific gravity table (Damas, LAKTA gravity separator) in a hull and a cotyledon/germ fraction. The cotyledons and germs were subsequently fractionated by retaining the cotyledons on a 2.25 mm split mesh sieve, and retaining the germs on a 1.5 mm round mesh sieve while passing a dust fraction through this latter sieve.

## Claims

1. Method for recovering a phytochemical from hulls of a seed, which method comprises fractionating a seed material comprising the hulls, thereby obtaining a fraction enriched in hulls and extracting the lignan from said fraction.

2. Method according to claim 1, wherein the fractionating encompasses fractionating based upon particle size.

3. Method according to claim 2, wherein the fractionating encompasses sieving, screening or a combination thereof.

4. Method according to any one of the preceding claims, wherein the fractionating encompasses fractionating based upon specific weight.

5. Method according to claim 4, wherein the fractionating based upon specific weight is carried out by employing at least one specific gravity table, at least one wind sifter or a combination thereof.

6. Method according to any one of the preceding claims, wherein the fraction enriched in hulls is identified by a visual evaluation, a specific weight evaluation or a combination thereof.

7. Method according to claim 6, wherein the fraction enriched in hulls has a specific weight in the range of 20-50 kg/hl, preferably in the range of 25-38 kg/hl, more preferably in the range of 25-30 kg/hl.

8. Method according to anyone of the preceding claims, wherein the phytochemical is extracted by liquid extraction, preferably by liquid extraction with water, a C₁-C₄ alcohol or a mixture thereof.

9. Method according to anyone of the preceding claims, comprising releasing or liberating at least part of the hulls in the seed material, preferably by rollermilling or hammermilling.

10. Method according to any one of the preceding claims, wherein the seed material is defatted.

11. Method according to any one of the preceding claims, wherein the seed material is a meal, preferably a coarse meal.

12. Method according to any one of the preceding claims, wherein the seed material is based upon at least one seed selected from the group consisting of *Linaceae, Poaceae, Brassicaceae, Arecaceae, Malvaceae Juglandaceae, Papilionaceae, Asteraceae, Pedaliaceae, Rosaceae, Apiaceae* and *Anacardiceae.*

13. Method according to any one of the preceding claims, wherein the phytochemical is a phenolic.

14. Method according to claim 13, wherein the phenolic is selected from the group consisting of lignans, flavonoids and stilbenes, preferably from the group of lignans.

15. Method according to claim 14, wherein the fraction enriched in hulls has a lignan content of more than 1.5 wt. % based upon the dry substance, preferably of more than 1.7 wt. % based upon the dry substance, more preferably of at least 2 wt % based upon the dry substance.

16. Fraction enriched in hulls obtainable by a method according to any one of the preceding claims.

17. Extract, obtainable by a method according to any one of the claims 1-15.

18. Extract according to claim 17, comprising 10-50 wt. %, based upon the dry weight, of the phytochemical, such as the lignan, and at least one carbohydrate, wherein the total carbohydrate to phytochemical weight to weight ratio is at least 0.2 .

19. Extract according to claim 17 or 18, comprising a solvent selected from the group consisting of water, C₁-C₄ alcohols and mixtures thereof.

20. Foodstuff, food supplement, cosmetic product, hair care product, body care product, animal feed, neutraceutical, cosmeceutical or medicament, comprising a fraction enriched in hulls according to claim 16 or an extract according to any one of the claims 17-19.
